# EUROPEAN PATENT APPLICATION

(11) **EP 4 442 313 A2**
(43) Date of publication of application: **09.10.2024**
(21) Application number: 22909925.4
(22) Date of filing: 19.12.2022
(51) Int. Cl.: A61N 5/10

(54) **RADIATION EXPOSURE SYSTEM, AND PLACEMENT PLATFORM CONTROL METHOD THEREFOR**

(30) Priority: 22.12.2021 CN 202111579574; 22.12.2021 CN 202111579834
(71) Applicant: Neuboron Therapy System Ltd., Xiamen, Fujian 361026 (CN)
(72) Inventor: GONG, Qiu-Ping, Nanjing, Jiangsu 211112 (CN); LIU, Yuan-hao, Nanjing, Jiangsu 211112 (CN)
(74) Representative: Gunzelmann, Rainer
(86) International application number: PCT/CN2022/139954
(87) International publication number: WO 2023/116612

(57) **Abstract**

Provided in the present invention are a radiation exposure system and a placement platform control method therefor. The radiation exposure system comprises a radiation generation device, a control device, a simulation positioning chamber, an irradiation chamber, as well as same first and second placement platforms that are respectively arranged in the simulation positioning chamber and the irradiation chamber, and same first and second placement platforms positioning devices that support the first and second placement platforms. An irradiated object has the same position on the first and second placement platforms. A simulated positioning position of the first placement platform is determined in the simulation positioning chamber by means of the first placement platform positioning device. The second placement platform positioning device is controlled by the irradiation indoor control device to move the second placement platform to the simulated positioning position and determine the irradiation position of the second placement platform. The irradiated object on the second placement platform is irradiated by a beam at the irradiation position. By simulated positioning in the simulation positioning chamber, the time for positioning the irradiated object in the irradiation chamber is saved, and the utilization rate of the irradiation chamber is increased.

## Description

### TECHNICAL FIELD

One aspect of the present invention relates to a radiation irradiation system, and another aspect of the present invention relates to a method for controlling a placement table of a radiation irradiation system.

### BACKGROUND

With the development of atomic science, radiation therapy, such as cobalt-60 radiation therapy, linear accelerator therapy and electron beam radiation therapy, has become one of the main means of cancer therapy. However, the traditional photon or electron therapy is limited by physical conditions of the radiation itself, which also damages a large amount of normal tissues in the beam path while killing tumor cells. In addition, due to different sensitivity of tumor cells to radiation, the traditional radiation therapy is often ineffective for therapy of malignant tumors with relatively high radiation resistance (e. g., glioblastoma multiforma and melanoma).

In order to reduce radiation damage to normal tissues surrounding tumors, the concept of target therapy in chemotherapy is applied to radiation therapy; for tumor cells with high radiation resistance, radiation sources with high relative biological effectiveness (RBE), such as proton therapy, heavy particle therapy and neutron capture therapy, are also actively developed at present. The neutron capture therapy combines the above two concepts, such as boron neutron capture therapy, which provides a better selection of cancer therapy than conventional radiation therapy by means of specific aggregation of a boron-containing drug in tumor cells, and in conjunction with precise neutron beam regulation.

In a radiation therapy process, a treatment table positioning device is used to enable the beam to be aligned with tumor cells in a object to be irradiated on the treatment table, so as to implement precise treatment, and minimize radiation damage to normal tissues surrounding the tumor cells to be irradiated. During neutron capture treatment, how to quickly and accurately position a treatment table and an object to be irradiated is related to the effect of therapy and the therapeutic comfort degree of a patient. During neutron capture treatment, the treatment table is located near the beam outlet, and a large amount of radiation remaining near the beam outlet will cause an additional radiation dose if the treatment table cannot be removed in time after the irradiation is completed.

Therefore, it is required to propose a new technical solution to solve the described problems.

### SUMMARY

In order to solve the described problems, an aspect of the present invention provides a radiation irradiation system, including a radiation generation device, a control device, a simulation positioning chamber, an irradiation chamber, and a first placement table and a second placement table which are identical and respectively provided in the simulation positioning chamber and the irradiation chamber, wherein, the radiation generation device is configured for generating beam and includes a beam outlet located within the irradiation chamber, the simulation positioning chamber is provided with a simulation beam outlet which is identical with the beam outlet, wherein a first placement table positioning device is further provided in the simulation positioning chamber, capable of supporting the first placement table, and carrying out a simulation positioning of the first placement table and an object to be irradiated on the first placement table; a second placement table positioning device is further provided in the irradiation chamber, capable of supporting the second placement table, and carrying out an irradiation positioning of the second placement table and an object to be irradiated on the second placement table; the first placement table positioning device and the second placement table positioning device are in an identical arrangement in the simulation positioning chamber and the irradiation chamber respectively, and are in an identical positional relationship with respect to the simulation beam outlet and the simulation beam outlet respectively; the control device is connected to the first positioning device and second placement table positioning device, and a placement location of the object to be irradiated on the first placement table is identical with a placement location of the object to be irradiated on the second table, a simulation positioning position for the first placement table is determined by the first placement table positioning device in the simulation positioning chamber, the control device is configured to control the second placement table positioning device to move the second placement table to the simulation positioning position and to determine an irradiation position of the second placement table in the irradiation chamber, and the object to be irradiated on the second placement table is irradiated by the beam at the irradiation position. Through the simulation positioning in the simulation positioning chamber, the time for positioning the object to be irradiated in the irradiation chamber is saved, and the utilization rate of the irradiation chamber is increased. The same placement table positioning device is used in the simulation positioning chamber and the irradiation chamber, so that the irradiation positioning is more convenient, quick and accurate.

Preferably, the radiation irradiation system further includes a treatment planning device, treatment plan data generated by the treatment planning device determines the placement location of the object to be irradiated on the first placement table in the simulation positioning chamber, the treatment planning device or the control device calculates treatment planning coordinate of the first placement table according to the treatment plan data and the placement location of the object to be irradiated, and the first placement table positioning device is controlled by the control device to move the first placement table to a treatment plan position determined by the treatment planning coordinate and determines the simulation positioning position of the first placement table. Through automatically calculating the coordinate of the treatment plan position of the placement table, and automatically controlling the placement table to move to the treatment plan position by the placement table positioning device, the accuracy and the speed of the positioning is high.

Further, laser positioning devices are provided in the simulation positioning chamber and the irradiation chamber respectively, the laser positioning devices are identical and in an identical positional relationship in the simulation positioning chamber and the irradiation chamber respectively, the object to be irradiated is provided with a marker corresponding to a position on the object to be irradiated where laser generated by the laser positioning device impinges on, and the object to be irradiated is determined based on the marker to locate at an identical position in the simulation positioning chamber and the irradiation chamber. By providing a laser positioning device, the positioning is more convenient and quicker.

Preferably, optical verification devices are provided in the simulation positioning chamber and the irradiation chamber respectively, the optical verification devices are identical and in an identical positional relationship in the simulation positioning chamber and the irradiation chamber respectively, the optical verification devices are capable of acquiring images of the object to be irradiated and a position of a placement table, transmitting data to a control module to compare with the treatment plan data, and adjusting or executing other treatment control in real time based on the compared result.

Preferably, the radiation irradiation system further includes a transferring vehicle and a transferring vehicle positioning device, the first placement table and the second placement table are a same placement table, the same placement table is capable of being supported and positioned by the transferring vehicle and transferred from the simulation positioning chamber to the irradiation chamber, and the transferring vehicle is fixed at identical starting positions in the simulation positioning chamber and the irradiation chamber respectively by the transferring vehicle positioning device.

Further, the radiation irradiation system further includes a placement table locking mechanism, at the identical starting positions, and the first placement table positioning device and the second placement table positioning device is controlled by the control device to connect to the same placement table and locked to the same placement table through the placement table locking mechanism.

Preferably, the radiation irradiation system is a neutron capture therapy system, the radiation generation device includes a neutron generation device and a beam shaping portion, wherein the beam shaping portion is capable of adjusting neutron beam generated by the neutron generation device to a pre-set beam quality, and the neutron beam generated by the neutron generation device irradiates the object to be irradiated on the placement table in the irradiation chamber through the beam shaping portion.

Further, the neutron generation device includes an accelerator and a target, charged particle beam generated by acceleration of the accelerator acts on the target to generate the neutron beam, and the beam shaping portion includes a reflecting portion, a retarding portion, a thermal neutron absorbing portion, a radiation shielding portion and a beam outlet, the retarding portion decelerates neutrons generated from the target to be in an epithermal neutron energy region, and the reflecting portion surrounds the retarding portion and directs deviating neutrons back to the retarding portion so as to improve intensity of the epithermal neutron beam, the thermal neutron absorbing portion is configured to absorb thermal neutrons so as to avoid excessive doses onto superficial normal tissues during treatment, and the radiation shielding portion is configured to shield photons and neutrons leaking from a portion other than the beam outlet.

A second aspect of the present invention provides a method for controlling a placement table of a radiation irradiation system, the radiation irradiation system includes a radiation generation device, a treatment planning device, a simulation positioning chamber, an irradiation chamber, and a first placement table and a second placement table which are identical and respectively provided in the simulation positioning chamber and the irradiation chamber, wherein a first placement table positioning device and a second placement table positioning device are respectively provided in the simulation positioning chamber and the irradiation chamber and capable of supporting the first placement table and the second table, wherein the first placement table positioning device and the second placement table positioning device have a same arrangement in the simulation positioning chamber and the irradiation chamber respectively, and in an identical positional relationship, the method for controlling the placement table includes: fixing and placing an object to be irradiated on the first placement table according to treatment plan data generated by the treatment planning device, and calculating a treatment plan position of the first placement table according to the treatment plan data and the placement location of the object to be irradiated; controlling the first placement table positioning device to move the first placement table to the treatment plan position and to determine a simulation positioning position; and placing the object to be irradiated on the second placement table as same on the first table, controlling the second placement table positioning device to move the second placement table to the simulation positioning position and to determine an irradiation position, and irradiating the object to be irradiated on the second placement table at the irradiation position by beam generated from the radiation generation device. Through the simulation positioning in the simulation positioning chamber, the time for positioning the object to be irradiated in the irradiation chamber is saved, and the utilization rate of the irradiation chamber is increased. Through automatically calculating the coordinate of the treatment plan position of the placement table, the same placement table positioning device is used in the simulation positioning chamber and the irradiation chamber to automatically control the movement of the loading placement table, so that the irradiation positioning is more convenient, quick and accurate.

Preferably, the radiation irradiation system further includes a transferring vehicle and a transferring vehicle positioning device, the first placement table and the second placement table are a same placement table, and the method for controlling the placement table further includes: before placing an object to be irradiated on the same placement table: fixing the transferring vehicle in the simulation positioning chamber by the transferring vehicle positioning device, and placing the same placement table on the transferring vehicle for positioning, so as to locate the same placement table at an initial position; and connecting and locking the first placement table positioning device to the same placement table in the simulation positioning chamber; after the simulation positioning position is determined: moving the same placement table to the initial position and supported by the transferring vehicle; unlocking the first placement table positioning device and the same placement table, and moving the same placement table and the object on the same placement table to the irradiation chamber; and placing the same placement table at the initial position in the irradiation chamber by the transferring vehicle positioning device, and connecting and locking the second placement table positioning device to the same placement table.

The radiation irradiation system and the method for controlling a placement table thereof of the present invention are provided, through the simulation positioning in the simulation positioning chamber, the time for positioning the object to be irradiated in the irradiation chamber is saved, and the utilization rate of the irradiation chamber is increased. The same placement table positioning device is used in the simulation positioning chamber and the irradiation chamber, so that the irradiation positioning is more convenient, quick and accurate.

A third aspect of the present invention provides a radiation irradiation system including a radiation generation device and a placement table, the radiation generation device is configured for generating beam and includes a beam outlet, and the beam passes through the beam outlet to irradiate an object to be irradiated on the placement table, wherein the radiation irradiation system further includes a placement table positioning device and a control device, the control device is connected to the placement table positioning device, the placement table is supported by a placement table positioning device and has an irradiation position and an ending position; at the irradiation position, the object to be irradiated is objected to irradiation with the beam on the table; at the ending position, the placement table moves away from the beam outlet; and the control device is capable of controlling the placement table positioning device to move the placement table from the irradiation position to the ending position after an irradiation of the beam is finished. After the irradiation is finished, the placement table is moved to a position away from the beam outlet, preventing the object to be irradiated from continuing to be irradiated by the residual radiation after the treatment is finished, and the unnecessary radiation dose is reduced.

Preferably, the placement table positioning device includes a robotic arm, the placement table is connected to and supported by the robotic arm, and the control device is capable of controlling the robotic arm such that a height of the placement table with respect to a ground changes from a height of the placement table with respect to the ground at the irradiated position after the irradiation of the beam is finished.

Further, the placement table positioning device further includes a linear shaft, the robotic arm is disposed between the linear shaft and the table, the placement table is connected to the linear shaft via the robotic arm, the placement table and the robotic arm is capable of translating together along the linear shaft, and the control device is capable of controlling the linear shaft to move the placement table from the irradiation position away from the beam outlet in an extending direction parallel to the linear shaft or controlling the robotic arm to move the placement table to a position of the placement table where the extending direction is substantially parallel to an extending direction of the linear shaft after the irradiation of the beam is finished.

Preferably, at the ending position, a minimum distance from the placement table to a plane perpendicular to a beam direction at which a center of the beam outlet is located is not less than 2500 mm, so as to ensure that the placement table is not exposed to a large dose of residual radiation. The height of a bearing surface of the placement table from a ground is not more than 600 mm, which facilitates the transferring of the object to be irradiated or the placement table.

Preferably, the control device includes a user interface, a system control module and a positioning control module, the user interface is connected to the system control module, and the system control module is connected to the positioning control module and the radiation generation device, the positioning control module is connected to the placement table positioning device and is capable of controlling a movement of the placement table positioning device, after reaching a predetermined irradiation time, the system control module is capable of controlling the radiation generation device to stop irradiating the object to be irradiated with the beam, and transmitting information to the user interface to indicate an ending state of treatment, and the system control module is capable of sending an instruction to the positioning control module to control the placement table positioning device to move the placement table from the irradiation position to the ending position after the treatment is completed.

Preferably, the radiation irradiation system is a neutron capture therapy system, the radiation generation device includes a neutron generation device and a beam shaping portion, wherein the beam shaping portion is capable of adjusting neutron beam generated by the neutron generation device to a pre-set beam quality, and the neutron beam generated by the neutron generation device irradiates the object to be irradiated on the placement table through the beam shaping portion.

Further, the neutron generation device includes an accelerator and a target, charged particle beam generated by acceleration of the accelerator acts on the target to generate the neutron beam, and the beam shaping portion includes a reflecting portion, a retarding portion, a thermal neutron absorbing portion, a radiation shielding portion and a beam outlet, the retarding portion decelerates neutrons generated from the target to be in an epithermal neutron energy region, and the reflecting portion surrounds the retarding portion and directs deviating neutrons back to the retarding portion so as to improve intensity of epithermal neutron beam, the thermal neutron absorbing portion is configured to absorb thermal neutrons so as to avoid excessive doses onto superficial normal tissues during treatment, and the radiation shielding portion is configured to shield photons and neutrons leaking from a portion other than the beam outlet.

A fourth aspect of the present invention provides a method for controlling a placement table of a radiation irradiation system, the radiation irradiation system includes a radiation generation device and a placement table, the radiation generation device is configured for generating beam and has a beam outlet, the beam passes through the beam outlet to irradiate an object to be irradiated on the placement table, wherein the radiation irradiation system further includes a placement table positioning device, the placement table is supported by the placement table positioning device and has an initial position, an irradiation position, and an ending position, the method for controlling the placement table includes: connecting and locking the placement table positioning device to the placement table carrying the object to be irradiated at the initial position; controlling the placement table positioning device to move the placement table to the irradiation position, and then starting to irradiate the object to be irradiated with the beam; and after the beam stops irradiating on the object to be irradiated, controlling the placement table positioning device to move the placement table to the ending position where the placement table moves away from the beam outlet. After the irradiation is finished, the placement table is moved to a position away from the beam outlet, thereby preventing the object to be irradiated from continuing to be irradiated by the residual radiation after the treatment is finished, and the unnecessary radiation dose is reduced.

Preferably, the placement table positioning device includes a linear shaft and a robotic arm, and a step of the controlling the placement table positioning device to move the placement table to the ending position includes: controlling the linear shaft to move the placement table away from the beam outlet in an extending direction of the linear shaft; controlling the robotic arm to move the placement table to a position of the placement table where an extending direction of the placement table is substantially parallel to an extending direction of the linear shaft; and controlling the robotic arm such that a height of the placement table with respect to a ground varies from a height of the placement table with respect to the ground at the irradiated position.

Preferably, the method for controlling the placement table may further include: after the beam starts to irradiate on the object to be irradiated, controlling the radiation generation device to stop the irradiation of the beam on the object to be irradiated and to indicate an ending state of treatment based on a predetermined irradiation time, and after the treatment is completed, controlling the placement table positioning device to move the placement table from the irradiation position to the ending position.

The present invention provides a radiation irradiation system and a method for controlling a loading placement table thereof. After the irradiation is completed, the placement table is moved to a position away from a beam outlet so as to preventing the object to be irradiated from continuing to be irradiated by the residual radiation after the treatment is finished, and the unnecessary radiation dose is reduced.

### BRIEF DESCRIPTION OF THE DRAWINGS

FIG. 1 is a schematic structural view of a neutron capture therapy system according to an embodiment of the present invention;
FIG. 2 is a schematic view of the positioning of a treatment table of a neutron capture therapy system according to an embodiment of the present invention;
FIG. 3 is a schematic structural view of a treatment table positioning device of the neutron capture therapy system according to an embodiment of the present invention;
FIG. 4 is a schematic view of the treatment table positioning device of FIG. 3 viewed in another direction;
FIG. 5 is a schematic block diagram of the neutron capture therapy system according to an embodiment of the present invention;
FIG. 6 is a schematic view of a treatment table transferring vehicle and a transferring vehicle positioning mechanism of the neutron capture therapy system according to an embodiment of the present invention;
FIG. 7 is a schematic view of the treatment table positioning device of the neutron capture therapy system at different positions according to an embodiment of the present invention;
FIG. 8 is a top view of the treatment table positioning device of FIG. 7 viewed in a direction parallel to the ground;
FIG. 9 is a sectional view of the treatment table positioning device of FIG. 8 taken from the plane of OO;
FIG. 10 is a flowchart of a method for controlling a treatment table of a neutron capture therapy system according to an embodiment of the present invention; and
FIG. 11 is a flowchart of a method for controlling a treatment table of a neutron capture therapy system to move away from a beam outlet according to an embodiment of the present invention.

### DETAILED DESCRIPTION OF THE EMBODIMENTS

Embodiments of the present invention are further described in detail below with reference to the accompanying drawings, so that those skilled in the art may implement the present invention according to the text of the specification.

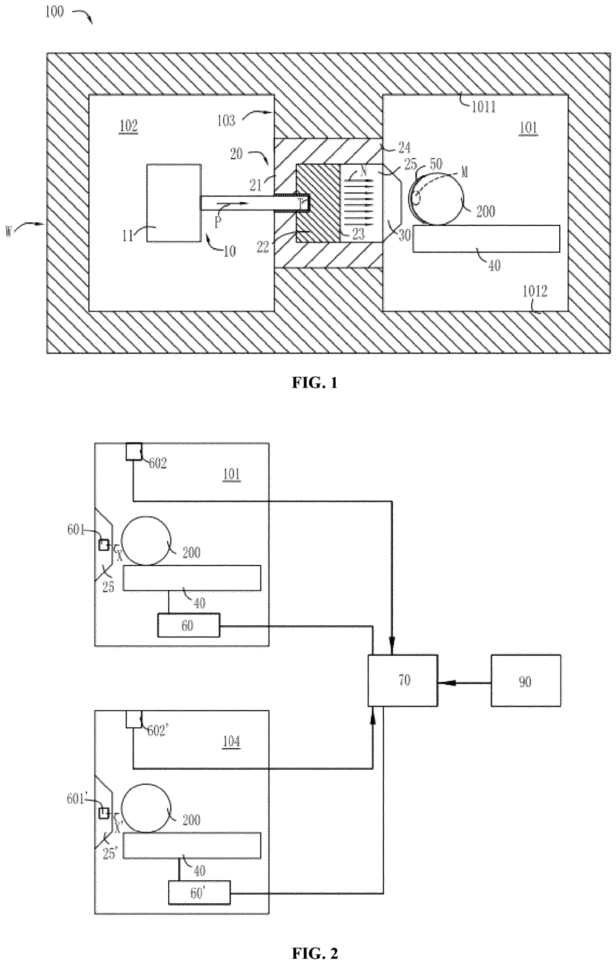

As shown in FIG. 1, a radiation irradiation system in the present embodiment is preferably a boron neutron capture therapy system 100, which includes a neutron generation device 10, a beam shaping portion 20, a collimator 30 and a treatment table 40. The neutron generation device 10 includes an accelerator 11 and a target T. The accelerator 11 accelerates charged particles (such as protons and deuteron) to generate charged particle beam P such as proton beam. The charged particle beam P irradiates the target T and act on the target T to generate neutron beam N. The target T is preferably a metal target. A suitable nuclear reaction is selected according to the required neutron yield and energy, energy and current of the available accelerated charged particles, and physical and chemical properties of the metal target, and the nuclear reactions commonly discussed are ⁷Li(p, n)⁷Be and ⁹Be(p, n)⁹B, which are both endothermic reactions. The energy threshold values of the two nuclear reactions are 1.881MeV and2.055MeV, respectively, and since the ideal neutron source for the boron neutron capture therapy is epithermal neutrons of keV energy level, and theoretically, if the Li target is bombarded with protons whose energy is only slightly higher than the energy threshold value, it is possible to generate neutrons of relatively low energy, which can be used in the clinic without too much slow speed treatment, however, the proton action cross sections of the two targets of lithium metal (Li) and beryllium metal (Be) with the threshold energy are not high, therefore protons of higher energy are usually chosen to initiate the nuclear reaction in order to generate a sufficiently large flux of neutrons. An ideal target have properties such as a high neutron yield, a distribution of energy of the generated neutron close to the energy region of the epithermal neutron (which will be described in detail below), no generation of too many long-distance radiation, low cost, safety, easy operation and high temperature resistance, but actually a nuclear reaction meeting all requirements cannot be found. A target made of lithium metal is used in embodiments of the present invention. However, it is well known to a person skilled in the art that the material of the target T may also be made of a metal material other than lithium and beryllium, such as tantalum (Ta) or tungsten (W). The target T may be a plate in a shape of a circle or other solid shapes, or may be in a liquid state (liquid metal). The accelerator 11 may be a linear accelerator, a cyclotron, a synchrotron and a synchrocyclotron. The neutron generation device 10 may also be a nuclear reactor without using an accelerator and a target. Whether the neutron source of the boron neutron capture therapy is generated from a nuclear reactor or the nuclear reaction of the accelerator charged particles with the target, a mixed radiation field is actually generated, i. e., the beam contains neutrons and photons with energy range from low energy to high energy. For the boron neutron capture therapy of deep tumors, in addition to epithermal neutrons, the greater the proportion of the remaining radiation rays, the greater the proportion of non-selective dose deposition in normal tissues, and therefore radiation of unnecessary doses should be reduced as much as possible. In addition, for the normal tissue of the object to be irradiated, excessive radiation which also results in unnecessary dose deposition is required to be avoided.

The neutron beam N generated by the neutron generation device 10 sequentially pass through the beam shaping portion 20 and the collimator 30 to irradiate the object to be irradiated 200 on the treatment table 40. The beam shaping portion 20 can adjust the beam quality of the neutron beam N generated by the neutron generation device 10.The collimator 30 is configured for converging the neutron beam N, so that the neutron beam N has a high degree of targeting in a process of treatment. The positions of the treatment table 40 and the object to be irradiated 200 may also be adjusted, so that the beam is aligned with the tumor cells M in the body of the object to be irradiated 200. These adjustments may be manually operated, or may be automatically implemented by a series of control mechanisms (described in detail below). It can be understood that the present invention may not have a collimator, and the beam coming out from the beam shaping portion 20directly irradiates the object to be irradiated 200 on the treatment table 40.

The beam shaping portion 20 further includes a reflecting portion 21, a retarding portion 22, a thermal neutron absorbing portion 23, a radiation shielding portion 24, and a beam outlet 25. Neutrons generated by the neutron generation device 10 have a wide energy spectrum, wherein in addition to the epithermal neutron meeting the therapy requirement, the proportion of other types of neutrons and photons needs to be minimized to avoid damage to the operator or the object to be irradiated, therefore, the neutrons bombarded out from the neutron generation device 10 need to pass through the retarding portion22 to adjust the fast neutron energy (greater than 40keV) therein to the epithermal neutron energy region (0.5eV-40keV) and minimize thermal neutrons (less than 0.5eV), the retarding portion22 is made of a material having a larger action cross section with the fast neutron and a smaller action cross section with epithermal neutron. As a preferred embodiment, the retarding portion22 is made of at least one of D₂O, AlF₃, Fluental^{™}, CaF₂, Li₂CO₃, MgF₂ and Al₂O₃. The reflecting portion21 surrounds the retarding portion22, reflects neutrons passing through the retarding portion22 and diffusing around back into the neutron beam N so as to improve the utilization rate of neutrons, and is made of a material having strong neutron-reflecting capacity. As a preferred embodiment, the reflecting portion21 is made of at least one of Pb and Ni. The thermal neutron absorbing portion23 is arranged behind the retarding portion22, and is made of a material having a large action cross section with the thermal neutron. As a preferred embodiment, the thermal neutron absorbing portion23 is made of Li-6,the thermal neutron absorbing portion23 is configured for absorbing the thermal neutrons passing through the retarding portion22, so as to reduce the proportion of the thermal neutrons in the neutron beam N, thus avoiding excessive doses from being applied to superficial normal tissues during the therapy. It is understandable that the thermal neutron absorbing portion may be integrated with the retarding portion, the material of the retarding portion contains Li-6. The radiation shielding portion24 is configured for shielding neutrons and photons leaked from parts other than the beam outlet 25. The material of the radiation shielding portion24 includes at least one of photon shielding material and neutron shielding material. As a preferred embodiment, the material of the radiation shielding portion24 includes photon shielding material plumbum (Pb) and neutron shielding material polyethylene (PE). The collimator 30 is arranged behind the beam outlet 25, the epithermal neutron beam coming out from the collimator 30 irradiates the object to be irradiated 200, passes through the superficial normal tissues, and then is retarded to thermal neutrons to reach the tumor cells M. It is understandable that the beam shaping portion 20 can have other configurations, as long as a epithermal neutron beam required for the therapy can be obtained. For convenience of description, when the collimator 30 is provided, an outlet of the collimator 30 may also be regarded as the beam outlet 25 described below.

Upon taking or injecting the boron-containing (B-10) drug into the object to be irradiated 200, the boron-containing drug selectively accumulates in tumor cells M. Based on a high capture cross section property of the boron-containing (B-10) drug for thermal neutrons, two heavy charged particles of ⁴He and ⁷Li are generated by a neutron capture and nuclear fission reaction of ¹⁰B(n, α)⁷Li. The average energy of the two charged particles of ⁴He and ⁷Li is about 2.33MeV, and the two charged particles have high Linear Energy Transfer (LET) and short distance properties, wherein the Linear Energy Transfer and distance of the alpha particle are 150keV/µm and 8µm, respectively, while that of the heavy particle of ⁷Li are 175keV/µm and 5µm,respectively, the total distance of the two heavy particles is approximately equal to a cell size, and therefore the radiation damage to organisms is limited to the cell level, which can achieve the purpose of locally killing tumor cells without causing too much damage to normal tissues.

In this embodiment, a radiation shielding device 50 is also provided between the object to be irradiated 200 and the beam outlet 25, and shields the radiation of the beam coming out of the beam outlet 25 to the normal tissues of the object to be irradiated. It can be understood that the radiation shielding device 50 may not be provided. The boron neutron capture therapy system 100 is housed entirely within a concrete-constructed building. Specifically, the boron neutron capture therapy system 100 further includes an irradiation chamber 101 and a charged particle beam generation chamber 102.The object to be irradiated 200 on the treatment table 40 is objected to therapy from the irradiation of the neutron beam N in the irradiation chamber 101.The charged particle beam generation chamber 102 at least partially houses the accelerator 11. The beam shaping portion 20 is at least partially housed within the partition wall 103 between the irradiation chamber 101 and the charged particle beam generation chamber 102. It can be understood that the partition wall 103 may completely separate the irradiation chamber 101 from the charged particle beam generation chamber 102. Alternatively, the irradiation chamber 101 and the charged particle beam generation chamber 102 may be partially separated from each other, and the irradiation chamber 101 and the charged particle beam generation chamber 102 are communicated with each other. One or more targets T may be provided, and the charged particles P may optionally act on one or more of the targets T or with all targets T to generate one or more therapeutic neutron beams N. Corresponding to the number of targets T, there may also be one or more beam shaping portions 20, one or more collimators 30 and one or more treatment tables40. The plurality of treatment tables may be disposed in a same irradiation chamber, or each treatment table may be provided in a separate irradiation chamber. The irradiation chamber 101 and the charged particle beam generation chamber 102 are spaces enclosed by concrete walls W (including the partition wall 103), and the concrete structure can shield neutrals and other radiation beams leaked during the operation of the boron neutron capture therapy system 100. Boron neutron capture therapy system 100 may also include a preparation chamber, a control chamber, and other spaces for assisting therapy (not shown). Each of the irradiation chambers may be provided with a preparation chamber for performing preparations such as injection of boron drugs and treatment plan simulation prior to irradiation therapy. The control chamber is configured for controlling the accelerator, a beam transmission portion, a treatment table positioning device, etc., and controlling and managing the whole irradiation process. A manager in the control chamber can also monitor a plurality of irradiation chambers at the same time. The boron neutron capture therapy system 100 can further include a simulation positioning chamber 104 (described in detail below) for performing simulation positioning of the object to be irradiated 200 prior to irradiation therapy. A simulation beam outlet 25' which is identical with the beam outlet 25 is provided in the simulation positioning chamber 104, thereby saving time for performing positioning of the object to be irradiated 200 in the irradiation chamber 101, and increasing the utilization rate of the irradiation chamber 101. It can be understood that the simulation positioning chamber can also be used as a preparation chamber.

Referring toFIG.2, the neutron capture therapy system 100 further includes a treatment table positioning device 60 and a control device 70, a treatment table 40 and the object to be irradiated 200 on the treatment table 40 are supported by the treatment table positioning device 60.Thecontrol device 70 is connected to the treatment table positioning device 60 and can control the treatment table positioning device 60. The control device 70 may also be connected to the neutron generation device 10 and may control the neutron generation device 10 to irradiate the neutron beam N onto the object to be irradiated 200 on the treatment table 40. In this embodiment, the irradiation chamber 101 and the simulation positioning chamber 104 are respectively provided with the same treatment table positioning devices 60, 60' which are in an identical positional relationship with respect to the beam outlet 25 and the simulation beam outlet 25'respectively. That is, the same operation coordinate system XYZ of the treatment table 40 and the treatment table positioning devices 60, 60' is defined in the irradiation chamber 101 and the simulation positioning chamber 104, and a reference point at a certain distance in the direction of the neutron beam N from the centers of the beam outlet 25 or the simulation beam outlet 25' is taken as the origin of coordinate. The treatment table positioning devices 60, 60' respectively perform simulation positioning and irradiation positioning of the treatment table 40 and the object to be irradiated 200 on the treatment table 40. By using the same treatment table positioning device, the irradiation positioning is more convenient, quick and accurate. For the convenience of description, only the structure of the treatment table positioning device 60 in the irradiation chamber 101 will be described in detail below.

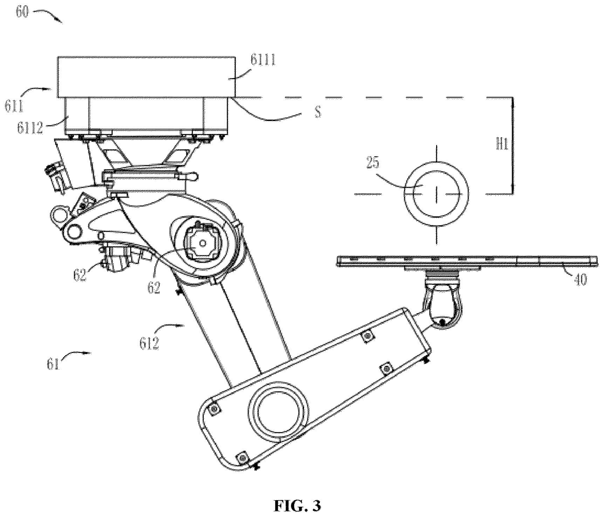

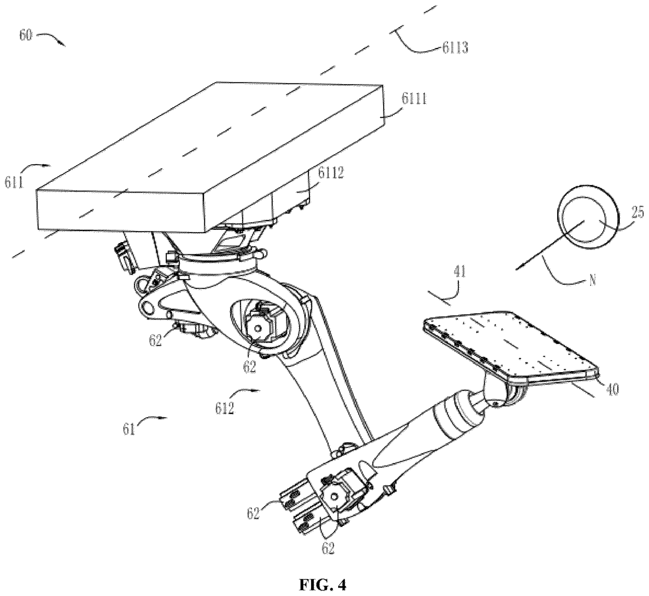

As shown in FIGS. 3-5, in one embodiment, the treatment table positioning device 60 includes a positioning mechanism 61.The positioning mechanism 61 includes a linear shaft 611 and a robotic arm 612, the robotic arm 612 is disposed between the linear shaft 611 and the treatment table 40, the treatment table 40 is connected to the linear shaft 611 via the robotic arm 612, and the treatment table 40 and the robotic arm 612 can be translated together along the linear shaft 611. In this embodiment, the linear shaft 611 is mounted to a ceiling 1011 of the irradiation chamber 101, and the robotic arm 612 extends as a whole towards a floor 1012 of the irradiation chamber 101. It can be understood that the linear shaft 611 may also be mounted to other surfaces, such as a wall or the floor. The linear shaft 611 is configured as a slide rail 6111 fixed to the ceiling 1011 and a support 6112 connected to the robotic arm 612, and the support 6112 can slide along the slide rail 6111. It can be understood that other configurations of the linear shaft are also possible. The linear shaft is directly fixed on the ceiling 1011, and a linear shaft fixing mechanism, such as a gantry frame of steel structure, is not additionally provided, so that the amount of steel used in an irradiation chamber is reduced, thereby avoiding secondary radiation caused by the activation of neutrons on the fixing mechanism. The robotic arm 612 is a multi-axis robotic arm connecting the support 6112 and the treatment table 40. The treatment table positioning device 60 further includes a driving mechanism 62 to drive the movement of the linear shaft 611 and the robotic arm 612. The driving mechanism 62 is controlled by the control device 70. An extending direction 6113 of the linear shaft 611 is parallel to the direction of the neutron beam N coming out from the beam outlet 25that is irradiated onto the object to be irradiated on the treatment table 40. Thus, in the process of the positioning of the treatment table, the robotic arm 612 as a whole is translated along a direction parallel to the direction of the neutron beam N, the robotic arm mainly is located in the space between the slide rail and the neutron beam outlet, reducing radioactivity generated by activating of neutrons on various parts of the robotic arms and the shortened service life caused thereby. The distance H1 from a slide surface S between the slide rail 6111 and the support 6112 to the center of the beam outlet 25 in the direction perpendicular to the slide surface S is less than 2 meters to provide sufficient operating space for the treatment table positioning device 60 to position the treatment table 40 at a desired position relative to the beam outlet 25. In this embodiment, the sliding surface S is parallel to the plane where the ceiling is located. It can be understood that the treatment table positioning device 60 may also have other arrangements, for example, a linear shaft 611 is not included, and the treatment table 40 is connected to and supported by the robotic arm 612; or the robotic arm 612 includes more or fewer arms.

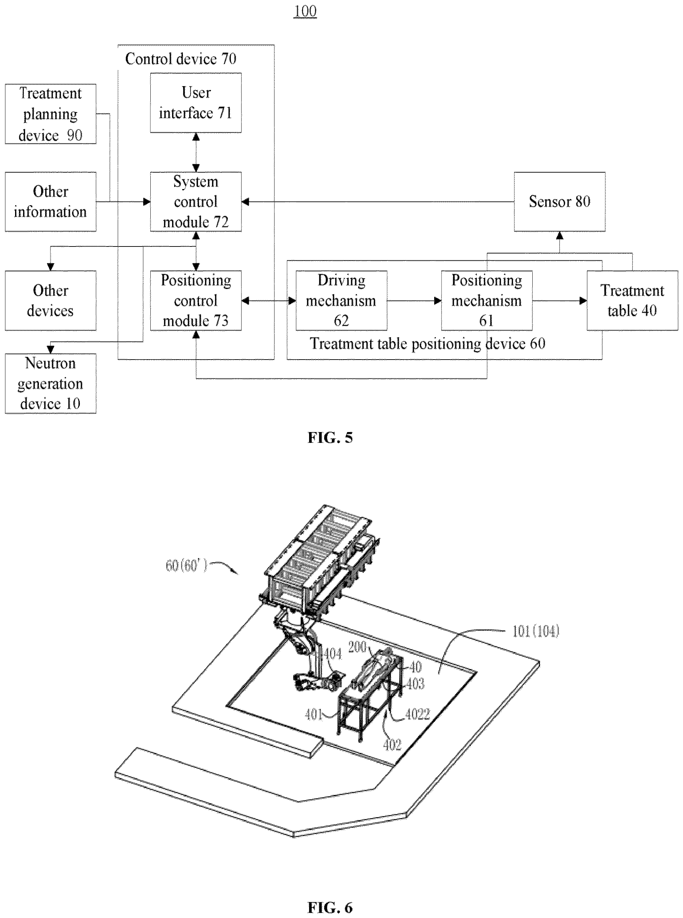

A sensor 80 may be disposed on the treatment table 40 or the treatment table positioning device 60. As shown in FIG. 5, the sensor 80 is disposed on the positioning mechanism 61 and the treatment table 40. In one embodiment, the sensor 80 is an anti-collision sensor disposed on the treatment table 40 and the robotic arm 612, when the edge of the treatment table or the robotic arm comes into contact with other objects or other objects reach the set range of the sensor, the sensor is triggered to send a signal and transmit same to the control device 70, and the control device 70 controls the driving mechanism 62 to stop driving the positioning mechanism 61 to move, that is, controls the treatment table 40 to stop moving. The anti-collision sensor may be a mechanical sensor, a photoelectric sensor, a radar sensor, an ultrasonic sensor, a laser range finder, etc.. It can be understood that the anti-collision sensor may also send a signal sensed by human body, and the operator may manually control the driving mechanism to stop driving based on the sensed signal; instead of controlling the treatment table to stop moving, other safety operations, such as the reversing movement prior to the collision, may be performed.

The control device 70 includes at least one user interface 71 allowing an operator to interactively engage in controlling the treatment table positioning device 60. The control device 70 further includes a system control module 72 and a positioning control module 73, wherein the user interface 71 is connected to the system control module 72, the system control module 72 is connected to the positioning control module 73, and the positioning control module 73 is connected to the driving mechanism 62 and controls the driving mechanism 62. When the system control module 72 receives an instruction sent by the user interface 71, the system control module 72 transmits the instruction to the positioning control module 73, and the positioning control module 73 automatically controls the movement of the positioning mechanism 61. Position information of the positioning mechanism 61 can be fed back to the system control module 72 by means of the positioning control module 73 and transmitted to the user interface 71 for indication of state. The operation state or data of the driving mechanism 62 is also fed back to the system control module 72 by means of the positioning control module 73, and the system control module 72 or the positioning control module 73 controls the driving mechanism 62 based on the information, and the system control module 72 may also transmit the information to the user interface 71 for indication of state. The sensor 80 is also connected to the system control module 72, and after receiving a signal sent by the sensor 80, the system control module 72 sends an instruction to the positioning control module 73 to control the movement of the treatment table positioning device 60, and transmits the sent by the sensor 80 to the user interface 71 for indication of state. It can be understood that the system control module 72 and the positioning control module 73 may be integrated together, or have other hardware arrangements.

The neutron capture therapy system 100 further includes a treatment planning device 90, wherein the treatment planning device 90 performs simulation calculation of the dose and generates a treatment plan (for example, by using a Monte Carlo simulation program) based on parameters of the treatment neutron beam N generated by the neutron generation device 10 and medical image data of the site to be irradiated. The treatment plan can determine the location of the site to be irradiated relative to the neutron generation device 10 during the irradiation treatment and the corresponding irradiation time. The control device 70 (system control module 71) is connected to the treatment planning device 90 and receives treatment plan data, so as to control movement of the treatment table positioning devices 60, 60' and the neutron beam N generated by the neutron generation device 10 according to the treatment plan data.

Before the irradiation treatment starts, the object to be irradiated 200 is simulation-positioned in the simulation positioning chamber 104 according to a treatment plan predetermined by the treatment planning system 90. First, the treatment table positioning device 60' is connected to the treatment table 40, as shown in FIG. 6. In this embodiment, the treatment table 40 is placed on the treatment table transferring vehicle 401. The transferring vehicle 401 is positioned in the simulation positioning chamber 104 (or the irradiation chamber 101) by the transferring vehicle positioning mechanism 402. At least two holes 4021 (not shown) are provided on the ground of the simulation positioning chamber 104 (or the irradiation chamber 101), at least two pins 4022 are correspondingly provided in the transferring vehicle 401, and the pins 4022 is inserted into the holes 4021 respectively for positioning. It can be understood that the transferring vehicle 401 may also be positioned in other manners. The relative position where the treatment table 40 is placed on the transferring vehicle 401 is also determined, for example, the position of the treatment table 40 is limited by a limiting mechanism 403 (for example, a boss provided on the transferring vehicle), and therefore, when the treatment table 40 is placed on the positioned transferring vehicle 401, the position of the treatment table 40 relative to the simulation positioning chamber 104 (or the irradiation chamber 101) is determined. At this time, the treatment table 40 is placed at the initial position A (having an identical positional relationship with the irradiation chamber 101) in the simulation positioning chamber 104. The control device 70 controls the treatment table positioning device 60' within the simulation positioning chamber 104 to move to a position where the treatment table positioning device 60' can be connected to the treatment table 40, and controls the locking mechanism 404 to lock the treatment table positioning device 60' and the treatment table 40. The specific structure of the locking mechanism 404 (clamping assembly) will not be described in detail here, which is included in a patent application published May 04, 2011 with the Chinese Patent Publication NO. 112741967 A, entitled NEUTRAL CAPTURE TREATMENT SYSTEM, and the patent application is incorporated herein by reference in its entirety.

Then, the object to be irradiated 200 is placed on the treatment table 40, and is placed and fixed based on the position of the site to be irradiated relative to the neutron generation device 10 during the irradiation treatment determined according to the predetermined treatment plan. The treatment planning device 90 or the control device 70 calculates the coordinate position of the treatment table 40 determined by the treatment plan based on the placement location at this time. The relative positions of the object to be irradiated and the treatment table can be determined by scanning the object to be irradiated and the treatment table after being placed through CT, optical scan, etc., so as to calculate the coordinate of the treatment table determined by the treatment plan based on the position of the site to be irradiated relative to the neutron generation device 10during the irradiation treatment determined according to the treatment plan. It can be understood that the coordinate position of the treatment table 40 may also be calculated in other manners.

The control device 70 automatically controls the treatment table positioning device 60' to move the treatment table 40 from the initial position A to the coordinate position (treatment plan position B) based on the calculated coordinate. After the treatment table 40 moves to the coordinate position (treatment plan position B), the operator may further adjust the treatment table 40 to determine the simulation positioning position C through the user interface 71 as required. If an error occurs during the movement of the treatment table 40 and the treatment table positioning device 60', it is returned to recalculate the movement path of the treatment table positioning device 60' or regenerate the treatment plan. By automatically calculating the coordinate of the treatment plan position of the treatment table, and automatically controlling the movement of the treatment table to the treatment plan position by the treatment table positioning device, the accuracy of the positioning is high and the speed is high.

Next, an instruction of completing the simulation position is sent via a user interface 71, the control device 70 records the coordinate position at this time (the simulation positioning position C), and controls the treatment table positioning device 60 to return the treatment table 40 to the initial position A (the treatment table 40 is right placed on the positioned transferring vehicle 401).The control device 70 controls the locking mechanism 403 to unlock and release the treatment table 40, and controls the treatment table positioning device 60 to move to a position separated from the treatment bed 40 and release the transferring vehicle positioning mechanism 402, so as to transport the treating table 40 and the object to be irradiated 200 to the irradiation chamber 101 by the transferring vehicle 401.

After the treating table 40 and the object to be irradiated 200reach the radiation chamber 101, the radiation positioning starts. The transferring vehicle 401 is provided with the same transferring vehicle positioning mechanism 402 in the radiation chamber 101 as in the simulation positioning chamber 104. That is, the transferring vehicle 401 in the irradiation chamber can be positioned by the transferring vehicle positioning mechanism 402 at the identical fixed position in the simulation positioning chamber 104, then the treatment table positioning device 60 in the irradiation chamber 101 is controlled to move to a position (the initial position A) where the treatment table positioning device 60 can be connected to the treatment table 40, and the locking mechanism 403 is controlled to lock the treatment table positioning device 60 with the treatment table 40. The control device 70 controls, based on the coordinate position determined by the simulation positioning, the treatment table 40 to move to the simulation positioning position C, and can further adjust same via the user interface 71 as required, and after adjustment, the irradiation position D is determined. The operator exits the irradiation chamber 101 and releases the transferring vehicle positioning mechanism 402 to remove the transferring vehicle 401.The simulation positioning in the simulation positioning chamber 104 saves the working time for positioning the object to be irradiated 200 before the irradiation treatment in the irradiation chamber 101 is performed, and can perform the irradiation treatment on another object to be irradiated while performing the simulation positioning, thereby increasing the utilization rate of the device. In one embodiment, when the treatment table 40 needs to move from the initial position A after the treatment table positioning devices 60, 60' are locked with the treatment table 40, the treatment table positioning devices 60, 60' may be controlled to lift the treatment table 40 first, and then release the transferring vehicle positioning mechanism 402 to remove the transferring vehicle 401, and then move further, thereby avoiding the treatment table positioning devices 60, 60' from positional interference with the transferring vehicle 401.

In one embodiment, laser positioning devices 601, 601' are identical and provided in the same position in both the irradiation chamber 101 and the simulation positioning chamber 104. The operator can mark the object to be irradiated 200 based on the position on the object to be irradiated 200 where the laser generated by the laser positioning device impinges on the object to be irradiated 200, and can adjust or verify the position of the object to be irradiated 200 in the simulation positioning chamber 104 and the irradiation chamber 101 based on the prepared mark, so as to ensure that the object to be irradiated 200 has the same position in the simulation positioning chamber 104 as that in the irradiation chamber 101. By providing laser positioning devices, the positioning is more convenient and quicker.

The laser generated by the laser positioning device 601, 601' can also determine the positions coinciding with the central axis X, X' of the beam outlet 25, 25', as shown in FIG.2, the position where the laser generated by the laser positioning devices 60, 60' impinges on the object to be irradiated 200 represents the position where the central axis of the beam coming out from the beam outlet 25, 25' impinges on the object to be irradiated 200. Marks are made on object to be irradiated 200 at the point of incidence of the voxel prosthesis tissue model on which the center axis of the beam simulated according to the treatment plan impinges, so that the incident position of the beam determined during the simulation positioning and the irradiation treatment is more accurate.

The optical verification devices 602 and 602'are provided in the irradiation chamber 101 and the simulation positioning chamber 104 respectively, and are identical and in an identical positional relationship. The optical verification devices are configured for acquiring images of the position of the treatment table 40 and the object to be irradiated 200, transmitting the data to the system control module 72 to compare the data with information such as a treatment plan, and adjusting or executing other treatment control based on the compared result. The system control module 72 may also receive other data information, such as data of the neutron generation device and information of the object to be irradiated, and control other devices such as the neutron generation device.

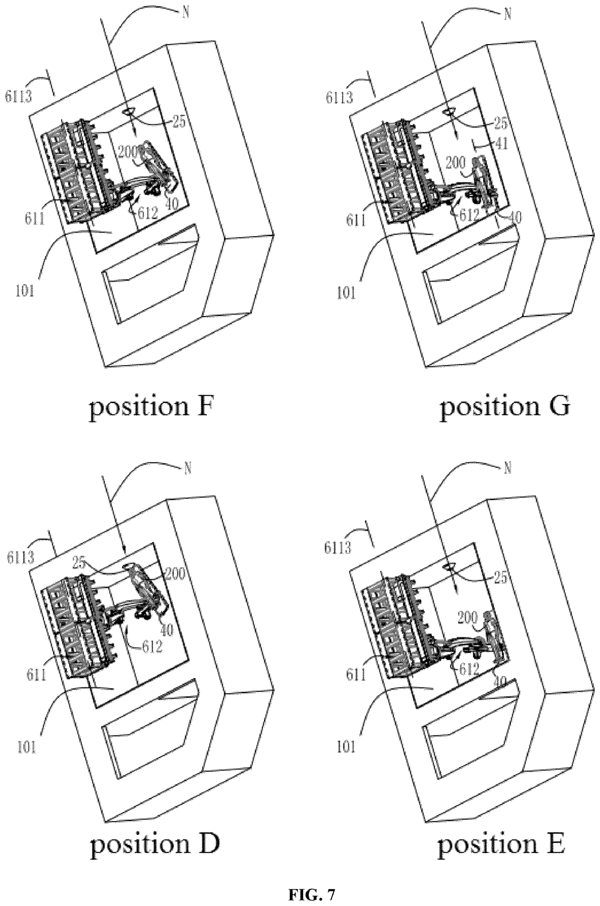

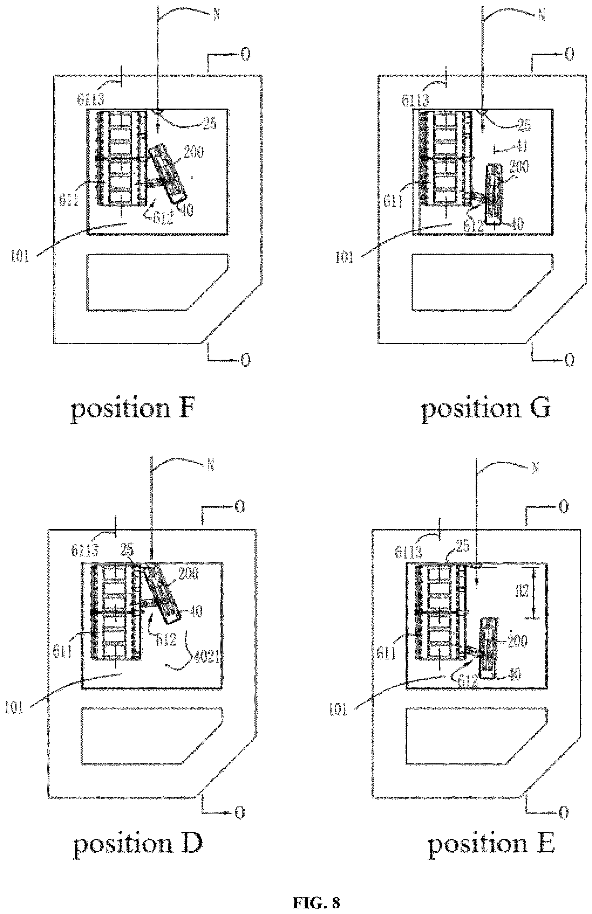

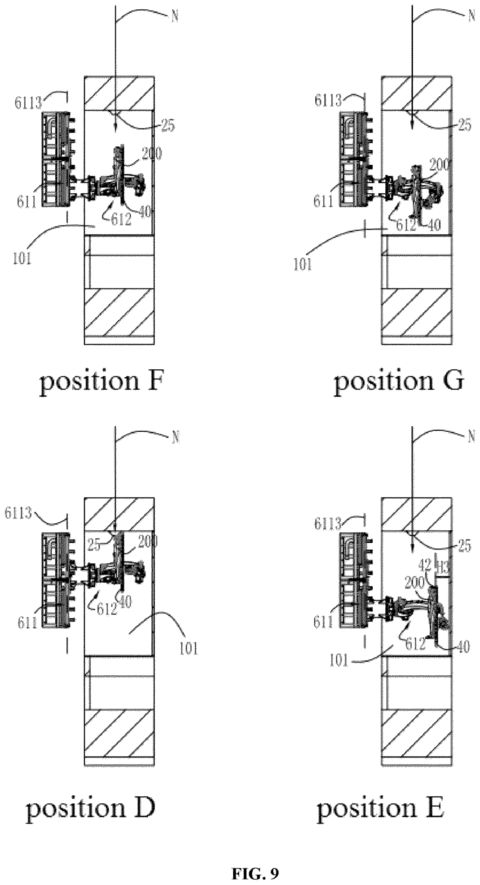

After the positions of the treatment table 40 and the obj ect to be irradiated 200 are adjusted, at this time, the treatment table 40 is at the irradiation position D. The operator sends an instruction for starting irradiation via the user interface 71, and the system control module 72 controls the neutron generation device 10 to start generating a neutron beam N to perform irradiation treatment on the object to be irradiated 200 on the treatment table 40 after determining that the condition for starting irradiation has been satisfied. After reaching the predetermined irradiation time (for example, the irradiation time determined according to the treatment plan data), the system control module 72 controls the neutron generation device 10 to stop irradiating the neutron beam N onto the object to be irradiated 200 on the treatment table 40, sends information to the user interface 71 to indicate an ending state of treatment, and then sends an instruction to the positioning control module 73 to control the treatment table positioning device 60 to move the treatment table 40 from the irradiation position D to the ending position E, so that the treatment table 40 moves away from beam outlet 25. After the neutron beam N stops irradiating, a large amount of radiation remains at the beam outlet 25, and the treatment table 40 is moved to a position away from the beam outlet 25, thereby preventing the object to be irradiated 200 from continuing to be irradiated by the residual radiation after the treatment is completed, so as to reduce an unnecessary radiation dose. Referring to FIGS. 7-9, schematic diagrams of states of the treatment table 40 and the treatment table positioning device 60 at different positions after treatment is completed are shown. After the treatment is completed, firstly, the linear shaft 611 is controlled to enable the treatment table 40 to move, along an extending direction 6113 parallel to the linear shaft 611, from the irradiation position D away from the beam outlet 25 to the first intermediate position F, so as to enable the treatment table 40 to move away from the beam outlet 25 quickly, thereby maximally avoiding that the object to be irradiated 200 continues to be irradiated with residual radiation after the treatment is completed; and secondly, the robotic arm 612 is controlled to enable the treatment table 40 to move to a second intermediate position G in the extending direction 41 of the treatment table 40 substantially parallel to the extending direction 6113 of the linear shaft 611, so as to prevent the treatment table 40 from interfering with the transferring bed and the like or blocking the outlet of the shielding door of the irradiation chamber 101 at the ending position E, thereby providing convenience for the irradiated object to be irradiated 200 to leave the irradiation chamber 101; and finally, the robotic arm 612 is controlled to move the treatment table 40 near the ground from the second intermediate position G to the ending position E, which facilitates the exit of the object to be irradiated 200 from the irradiation chamber 101. After the object to be irradiated 200 leaves, the locking mechanism 403 can also be controlled to unlock, and the treatment table 40 is returned to the simulation positioning chamber 104, Alternatively, the locking mechanism 403 may be controlled to be unlocked and the object to be irradiated 200 may be taken out of the irradiation chamber 101 together with the treatment table 40. After the object to be irradiated 200 leaves from the treatment table 40, the treatment table 40 is returned to the simulation positioning chamber 104, such as by the transferring vehicle 401. In this case, the ending position E may be the same as the initial position A, and the transferring vehicle 401 is also positioned in the irradiation chamber 101 by the transferring vehicle positioning mechanism 402. The robotic arm 612 moves the treatment table 40 to the initial position A (the ending position E) where the treatment table 40 is placed right on the transferring vehicle 401. At the ending position E, the minimum distance H2 from the treatment table 40 to the plane at which the center of the beam outlet 25 is located perpendicular to the direction of the neutron beam N is not less than 2500 mm, ensuring that the treatment table is not irradiated by a large dose of residual radiation. At the ending position E, the height H3 of the support surface 42 of the treatment table 40 from the ground is not larger than 600 mm, thereby facilitating the transfer of the object to be irradiated 200 or treatment table 40.

After the treatment is completed, the system control module 72 may automatically control the treatment table 40 to move away from the beam outlet 25 based on the signal indicating the irradiation time has been reached or the signal indicating the stop of irradiation of the neutron beam N. Alternatively, an operator may input, on the user interface 71, an instruction for the treatment table to move away from the beam outlet based on the ending state of treatment shown in the user interface 71, for example, by clicking a corresponding key on the man-machine interactive control interface 713, the system control module 72 controls, based on the instruction, the treatment table 40 to move away from the beam outlet 25.

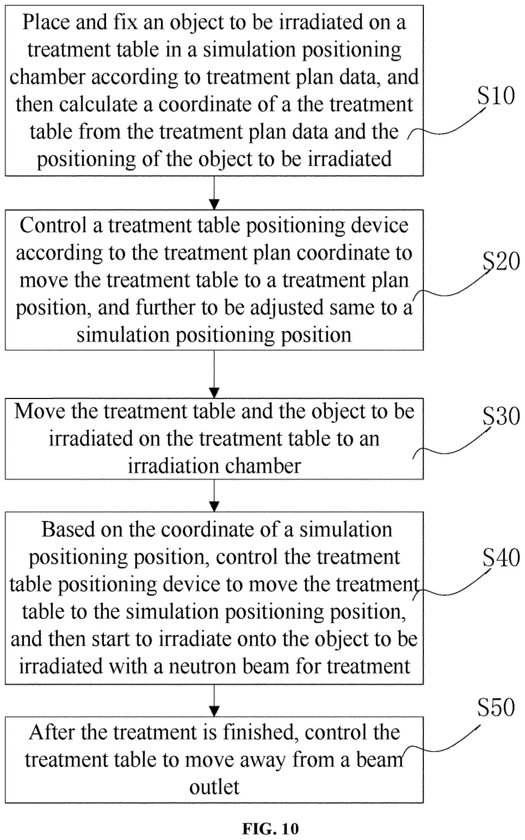

As shown in FIG. 10, in brief, a method for controlling the treatment table of this embodiment includes:
S10: The treatment table positioning device 60' in the simulation positioning chamber 104 is connected and locked to the treatment table 40 in the simulation positioning chamber 104, the object to be irradiated 200is placed and fixed on the treatment table 40 according to treatment plan data, and then a treatment plan coordinate of the treatment table 40, i. e. a coordinate of a treatment plan position B, is calculated according to the treatment plan data and the positioning of the object to be irradiated 200.
S20: The treatment table positioning device 60' is controlled according to the treatment plan coordinate to move the treatment table 40 to the treatment plan position B, and further adjust the treatment table 40 to the simulation positioning position C as required, and the coordinate of the simulation positioning position C is recorded. It can be understood that the simulation positioning position C may also be the treatment plan position B.
S30: The treatment table positioning device 60' is unlocked from the treatment table 40, the treatment table 40 and the object to be irradiated 200 on the treatment table 40 are moved to the irradiation chamber 101, and the treatment table positioning device 60 in the irradiation chamber 101 is connected and locked to the treatment table 40.
S40: Based on the coordinate of the simulation positioning position C, the treatment table positioning device 60 is controlled to move the treatment table 40 to the simulation positioning position C, and further adjust the treatment table 40to the irradiation position D as required, and then the neutron beam N starts to irradiate onto the object to be irradiated 200for treatment. It can be understood that the irradiation position D may also be the simulation positioning position C.
S50: After the treatment is finished, that is, after the neutron beam N stops irradiating onto the object to be irradiated 200, the treatment table positioning device 60 is controlled to move the treatment table 40 to the ending position E, that is, the treatment table 40 is controlled to move away from the beam outlet 25.

It can be understood that S20 may further include: if an error occurs during the movement of the treatment table 40 to the treatment plan position B, it is returned to recalculate the movement path of the treatment table positioning device 60' or regenerate the treatment plan.

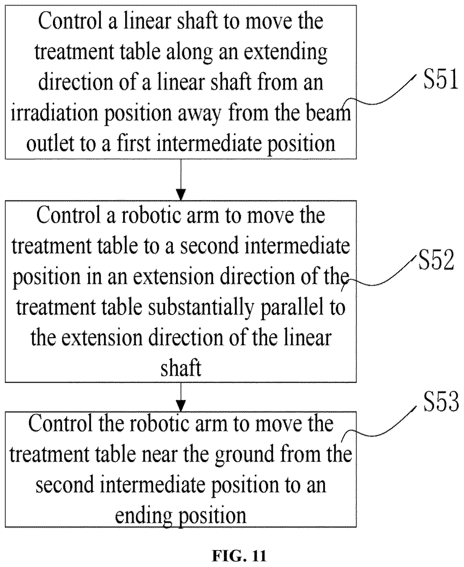

As shown in FIG 11, specifically, the method for controlling the treatment table 40 to move away from the beam outlet 25 in S50 further includes:
S51: The linear shaft 611 is controlled to move the treatment table 40 along an extending direction 6113 parallel to the linear shaft 611 from the irradiation position D away from the beam outlet 25 to a first intermediate position F.
S52: The robotic arm 612 is controlled to move the treatment table 40 to the second intermediate position G in the extension direction 41 of the treatment table 40 substantially parallel to the extension direction 6113 of the linear shaft 611.
S53: The robotic arm 612 is controlled to move the treatment table 40 near the ground from the second intermediate position G to the ending position E.

The instruction input on the user interface 71 that the treatment table moves away from the beam outlet may be automatic continuous execution of S51-S53 through one button or stepwise execution of S51-S53 through three buttons respectively corresponding to S51-S53. Other arrangement manners may also be available. It may be understood that, S51 and S53 may also be: the robotic arm 612 is firstly controlled to move the treatment table 40 near the ground, and then the linear shaft 611 is controlled to move the treatment table 40 to away from the beam outlet 25 along an extending direction 6113 parallel to the linear shaft 611, and S52 may also be performed after S53. Alternatively, the linear shaft 611 and the robotic arm 612 are controlled simultaneously to move the treatment table 40 to the ending position E. It can be understood that, according to specific requirements, the height of the table with respect to the ground at the ending position E may also be other positions where the height of the placement table relative to the ground changes as compared with the height of the placement table relative to the ground at the irradiation position D.

The above-mentioned positions A-G are all based on a reference point pre-set on the treatment table 40. It can be understood that a transferring vehicle may also not be provided, the simulation positioning chamber and the irradiation chamber are respectively provided with the same treatment tables, and the object to be irradiated is objected to the same positioning in the simulation positioning chamber and the irradiation chamber (for example, by a positioning mechanism provided on the treatment table), and the same initial positions are determined(for example, by laser positioning devices).

The concrete wall in this embodiment is a wall made of boron-containing heavy spar concrete having a thickness of 1 m or more and a density of 3 g/c. c.. The boron-containing concrete has better absorption performance of neutrons, and can also reduce the amount of neutron exposure to metal materials in the concrete in addition to enhancing the radiation shielding effect of the concrete. It will be appreciated that other thicknesses or densities may be provided or substituted for other materials and that the thickness, density or material of different portions of the concrete wall may be different. It can be understood that the present invention can also be applied to other types of neutron irradiation systems, and can also be applied to other radiation irradiation systems, such as proton treatment systems and heavy ion treatment systems. In this case, the neutron generation device can be replaced with other radiation generation devices, and the material of the concrete can be replaced as required. The treatment table may be a placement table of another object to be irradiated.

Although the illustrative embodiments of the present invention have been described above to facilitate understanding of the present invention by those skilled in the art, however, it should be clear that the present invention is not limited to the specific embodiments, as long as such variations are within the spirit and scope of the invention as defined and determined by the appended claims, these variations are obvious and are within the scope of the present invention.

## Claims

1. A radiation irradiation system, comprising a radiation generation device, a control device, a simulation positioning chamber, an irradiation chamber, and a first placement table and a second placement table which are identical and respectively provided in the simulation positioning chamber and the irradiation chamber, wherein, the radiation generation device is configured for generating beam and comprises a beam outlet located within the irradiation chamber, the simulation positioning chamber is internally provided with a simulation beam outlet which is identical with the beam outlet,
**characterized in that** a first placement table positioning device is arranged in the simulation positioning chamber, capable of supporting the first placement table and carrying out a simulation positioning of the first placement table and an object to be irradiated on the first placement table; a second placement table positioning device is arranged in the irradiation chamber, capable of supporting the second placement table and carrying out an irradiation positioning of the second placement table and an object to be irradiated on the second placement table; the first placement table positioning device and the second placement table positioning device are in an identical arrangement in the simulation positioning chamber and the irradiation chamber respectively, and are in an identical positional relationship with respect to the simulation beam outlet and the simulation beam outlet respectively; the control device is connected to both the first positioning device and the second placement table positioning device, and a placement location of the object to be irradiated on the first placement table is identical with a placement location of the object to be irradiated on the second placement table, a simulation positioning position of the first placement table is determined by the first placement table positioning device in the simulation positioning chamber, the control device is configured to control the second placement table positioning device to move the second placement table to the simulation positioning position and to determine an irradiation position of the second placement table in the irradiation chamber, and the object to be irradiated on the second placement table is irradiated by the beam at the irradiation position.

2. The radiation exposure system according to claim 1, further comprising a treatment planning device, wherein treatment plan data generated by the treatment planning device determines a placement location of the object to be irradiated on the first placement table in the simulation positioning chamber, the treatment planning device or the control device calculates treatment planning coordinate of the first placement table according to the treatment plan data and the placement location of the object to be irradiated on the first placement table, and the control device controls the first placement table positioning device to move the first placement table to a treatment plan position determined by the treatment planning coordinate and determines the simulation positioning position of the first placement table.

3. The radiation irradiation system according to claim 2, wherein laser positioning devices are arranged in the simulation positioning chamber and the irradiation chamber respectively, the laser positioning devices are identical and in an identical positional relationship in the simulation positioning chamber and the irradiation chamber respectively, the object to be irradiated is provided with a marker corresponding to a position on the object to be irradiated where laser generated by any one of the laser positioning devices impinges on, and the object to be irradiated is determined based on the marker to locate at an identical position in the simulation positioning chamber and the irradiation chamber.

4. The radiation irradiation system according to claim 1, wherein optical verification devices are provided in the simulation positioning chamber and the irradiation chamber respectively, the optical verification devices are identical and in an identical positional relationship in the simulation positioning chamber and the irradiation chamber respectively, the optical verification devices are capable of acquiring images of the object to be irradiated and positions of the first placement table and the second placement table, and transmitting data to a control module to compare with the treatment plan data.

5. The radiation irradiation system according to claim 1, further comprising a transferring vehicle and a transferring vehicle positioning device, wherein the first placement table and the second placement table are a same placement table, the placement table is capable of being supported and positioned by the transferring vehicle and transferred from the simulation positioning chamber to the irradiation chamber, and the transferring vehicle is fixed at identical starting positions in the simulation positioning chamber and the irradiation chamber respectively by the transferring vehicle positioning device.

6. The radiation irradiation system according to claim 5, further comprising a placement table locking mechanism, wherein at the identical starting positions, the first placement table positioning device and the second placement table positioning device are controlled by the control device to connect to the same placement table and locked to the same placement table by the placement table locking mechanism.

7. The radiation irradiation system according to claim 1, wherein the radiation irradiation system is a neutron capture therapy system, the radiation generation device comprises a neutron generation device and a beam shaping portion, wherein the beam shaping portion is capable of adjusting a neutron beam generated by the neutron generation device to a pre-set beam quality, and the neutron beam generated by the neutron generation device irradiates the object to be irradiated on the placement table in the irradiation chamber through the beam shaping portion.

8. The radiation irradiation system according to claim 7, wherein the neutron generation device comprises an accelerator and a target, a charged particle beam generated by acceleration of the accelerator acts on the target to generate the neutron beam, and the beam shaping portion comprises a reflecting portion, a retarding portion, a thermal neutron absorbing portion, a radiation shielding portion and the beam outlet, the retarding portion decelerates neutrons generated from the target to be in an epithermal neutron energy region, and the reflecting portion surrounds the retarding portion and directs deviating neutrons back to the retarding portion so as to improve intensity of an epithermal neutron beam, the thermal neutron absorbing portion is configured to absorb thermal neutrons so as to avoid excessive doses onto superficial normal tissues during treatment, and the radiation shielding portion is configured to shield photons and neutrons leaking from a portion other than the beam outlet.

9. A method for controlling a placement table of a radiation irradiation system, wherein the radiation irradiation system comprises a radiation generation device, a treatment planning device, a simulation positioning chamber, an irradiation chamber, and a first placement table and a second placement table which are identical and respectively arranged in the simulation positioning chamber and the irradiation chamber,
**characterized in that** a first placement table positioning device and a second placement table positioning device are respectively arranged in the simulation positioning chamber and the irradiation chamber and capable of supporting the first placement table and the second placement table, wherein the first placement table positioning device and the second placement table positioning device are in an identical arrangement and in an identical positional relationship in the simulation positioning chamber and the irradiation chamber respectively, the method for controlling the placement table comprises:
fixing and placing an object to be irradiated on the first placement table according to treatment plan data generated by the treatment planning device, and calculating a treatment plan position of the first placement table according to the treatment plan data and a placement location of the object to be irradiated;
controlling the first placement table positioning device to move the first placement table to the treatment plan position and determining a simulation positioning position; and
placing the object to be irradiated on the second placement table as same as on the first placement table, controlling the second placement table positioning device to move the second placement table to the simulation positioning position and determining an irradiation position, and irradiating the object to be irradiated on the second placement table at the irradiation position by a beam generated by the radiation generation device.

10. The method for controlling the placement table according to claim 9, wherein the radiation irradiation system further comprises a transferring vehicle and a transferring vehicle positioning device, the first placement table and the second placement table are a same placement table, and the method for controlling the placement table further comprises:
before placing an object to be irradiated on the same placement table:
fixing the transferring vehicle in the simulation positioning chamber by the transferring vehicle positioning device, and placing the same placement table on the transferring vehicle for positioning, so as to locate the same placement table at an initial position; and
connecting and locking the first placement table positioning device to the same placement table in the simulation positioning chamber;
after determining a simulation positioning position:
moving the same placement table to the initial position and supporting the same placement table by the transferring vehicle;
unlocking the first placement table positioning device and the same placement table, and moving the same placement table and the object to be irradiated on the same placement table to the irradiation chamber; and
placing the same placement table at the initial position in the irradiation chamber by the transferring vehicle positioning device, and connecting and locking the second placement table positioning device to the same placement table.

11. A radiation irradiation system comprising a radiation generation device and a placement table, the radiation generation device being configured for generating a beam and comprising a beam outlet, and the beam passing through the beam outlet to irradiate an object to be irradiated on the placement table,
**characterized in that** the radiation irradiation system further comprises a placement table positioning device and a control device, the control device is connected to the placement table positioning device, the placement table is supported by the placement table positioning device and has an irradiation position and an ending position; at the irradiation position, the object to be irradiated is objected to irradiation of the beam on the placement table; at the ending position, the placement table moves away from the beam outlet; and the control device is capable of controlling the placement table positioning device to move the placement table from the irradiation position to the ending position after the irradiation of the beam is finished.

12. The radiation irradiation system according to claim 1, wherein the placement table positioning device comprises a robotic arm, the placement table is connected to and supported by the robotic arm, and the control device is capable of controlling the robotic arm such that a height of the placement table with respect to a ground changes from a height of the placement table with respect to the ground at the irradiated position after the irradiation of the beam is finished.

13. The radiation irradiation system according to claim 2, wherein the placement table positioning device further comprises a linear shaft, the robotic arm is disposed between the linear shaft and the placement table, the placement table is connected to the linear shaft via the robotic arm, the placement table and the robotic arm is capable of translating together along the linear shaft, and the control device is capable of controlling the linear shaft to move the placement table from the irradiation position away from the beam outlet in an extending direction parallel to the linear shaft or controlling the robotic arm to move the placement table to a position where an extending direction of the placement table is substantially parallel to an extending direction of the linear shaft after the irradiation of the beam is finished.

14. The radiation irradiation system according to claim 1, wherein at the ending position, a minimum distance from the placement table to a plane perpendicular to a beam direction at which a center of the beam outlet is located is not less than 2500 mm, and a height of a bearing surface of the placement table from a ground is not more than 600 mm.

15. The radiation irradiation system according to claim 1, wherein the control device comprises a user interface, a system control module and a positioning control module, the user interface is connected to the system control module, and the system control module is connected to the positioning control module and the radiation generation device, the positioning control module is connected to the placement table positioning device and is capable of controlling a movement of the placement table positioning device, after reaching a predetermined irradiation time, the system control module is capable of controlling the radiation generation device to stop the irradiation of the beam on the object to be irradiated, and transmitting information to the user interface to indicate an ending state of treatment, and the system control module is capable of sending an instruction to the positioning control module to control the placement table positioning device to move the placement table from the irradiation position to the ending position after the treatment is completed.

16. The radiation irradiation system according to claim 1, wherein the radiation irradiation system is a neutron capture therapy system, the radiation generation device comprises a neutron generation device and a beam shaping portion, wherein the beam shaping portion is capable of adjusting a neutron beam generated by the neutron generation device to a pre-set beam quality, and the neutron beam generated by the neutron generation device irradiates the object to be irradiated on the placement table through the beam shaping portion.

17. The radiation irradiation system according to claim 6, wherein the neutron generation device comprises an accelerator and a target, a charged particle beam generated by acceleration of the accelerator acts on the target to generate the neutron beam, and the beam shaping portion comprises a reflecting portion, a retarding portion, a thermal neutron absorbing portion, a radiation shielding portion and the beam outlet, the retarding portion decelerates neutrons generated from the target to be in an epithermal neutron energy region, and the reflecting portion surrounds the retarding portion and directs deviating neutrons back to the retarding portion so as to improve intensity of an epithermal neutron beam, the thermal neutron absorbing portion is configured to absorb thermal neutrons so as to avoid excessive doses onto superficial normal tissues during the treatment, and the radiation shielding portion is configured to shield photons and neutrons leaking from a portion other than the beam outlet.

18. A method for controlling a placement table of a radiation irradiation system, wherein the radiation irradiation system comprises a radiation generation device and a placement table, the radiation generation device is configured for generating a beam and has a beam outlet, the beam passes through the beam outlet to irradiate an object to be irradiated on the placement table,
**characterized in that** the radiation irradiation system further comprises a placement table positioning device, the placement table is supported by the placement table positioning device and has an initial position, an irradiation position, and an ending position, the method for controlling the placement table comprises:
connecting and locking the placement table positioning device to the placement table carrying the object to be irradiated at the initial position;
controlling the placement table positioning device to move the placement table to the irradiation position, and then starting to irradiate the object to be irradiated with the beam; and
after the beam stops irradiating on the object to be irradiated, controlling the placement table positioning device to move the placement table to the ending position where the placement table is away from the beam outlet.

19. The method for controlling the placement table according to claim 8, wherein the placement table positioning device comprises a linear shaft and a robotic arm, and a step of controlling the placement table positioning device to move the placement table to the ending position comprises:
controlling the linear shaft to move the placement table away from the beam outlet in an extending direction of the linear shaft;
controlling the robotic arm to move the placement table to a position where an extending direction of the placement table is substantially parallel to an extending direction of the linear shaft; and
controlling the robotic arm such that a height of the placement table with respect to a ground changes from a height of the placement table with respect to the ground at the irradiated position.

20. The method for controlling the placement table according to claim 8, further comprising:
after starting to irradiate the object to be irradiated with the beam, controlling the radiation generation device to stop the irradiation of the beam on the object to be irradiated and to indicate an ending state of treatment based on a predetermined irradiation time, and after the treatment is completed, controlling the placement table positioning device to move the placement table from the irradiation position to the ending position.
